# EUROPEAN PATENT APPLICATION

(11) **EP 2 266 515 A1**
(43) Date of publication of application: **29.12.2010**
(21) Application number: 09725051.8
(22) Date of filing: 26.03.2009
(51) Int. Cl.: A61F 13/32

(54) **APPLICATOR FOR TAMPON**

(30) Priority: 26.03.2008 JP 2008081950; 26.03.2008 JP 2008081964
(71) Applicant: Unicharm Corporation, Ehime 799-0111 (JP)
(72) Inventor: The designation of the inventor has not yet been filed
(74) Representative: Kensett, John Hinton
(86) International application number: PCT/JP2009/056074
(87) International publication number: WO 2009/119717

(57) **Abstract**

An applicator for a tampon comprises an outer cylinder (2) containing an absorber (4) therein and provided with a petal-like components (8) for pushing out the absorber (4) on one side thereof and with a grip cylinder (7) on the other side thereof, and an inner tube (3) which is inserted into the grip cylinder (7) and can push out the absorber (4) to the outside from the petal-like components (8) by moving into the outer cylinder (2). A plurality of rows of annular protrusions (11, 12, 13, 14) are formed in the whole area of the grip cylinder (7) in the circumferential direction, such that the height from the outer circumferential surface of the grip cylinder (7) decreases gradually toward the side of the inner tube (3) being inserted into the grip cylinder (7).

## Description

### TECHNICAL FIELD

The present invention relates to a tampon applicator housing an absorber and ejecting the absorber when it is used.

### BACKGROUND ART

A tampon applicator has an outer cylinder housing an absorber, and an inner cylinder ejecting the absorber housed in the outer cylinder. The outer cylinder and the inner cylinder are formed by injection molding of resin. The inner cylinder is handled so as to move into the outer cylinder. This handling acts to eject the absorber out from the outer cylinder. An ejection opening formed at the end of the outer cylinder is provided with petal-like components which are kept normally closed. The absorber pushed by the inner cylinder stretches the petal-like components, and is then ejected through the ejection opening out from the outer cylinder. In order to ensure an exact handling for moving the inner cylinder, the outer cylinder has a grip cylinder (grip) formed thereon. Published Literatures 1 to 4 disclose prior arts for improving the grip cylinder.

According to Patent Literature 1, the grip cylinder is provided with a flat surface. A high-friction structure is obtained by forming slits or the like on the flat surface. According to Patent Literature 2, the grip cylinder is configured to have a geometry recessed by 10 mm or smaller from the outer cylinder in the radial direction of the outer cylinder. According to Patent Literature 2, the recessed portion is provided with projections not as high as 10 mm. According to Patent Literature 3, the grip cylinder is provided with a flat portion having a geometry of nearly elliptic column. Rib-like projections are provided to the outer circumference of the flat portion. According to Patent Literature 4, the grip cylinder is configured to have a near-polygonal prism form, and is provided with rib-like projections on the outer circumference thereof.
Patent Literature 1: Japanese Patent Application Publication No. 2004-532712
Patent Literature 2: International Patent Application Publication WO 2005-0009312
Patent Literature 3: Japanese Patent Application Publication 61-54822
Patent Literature 4: Japanese Patent Application Publication 61-54823

All of the conventional tampon applicators described in the above have anti-slipping projections formed to the grip cylinders. Injection molding of those may, therefore, need die cutting or use of a segment die. In the general process of injection molding, the tampon applicator is released from the die by forced ejection, because the portion of the grip cylinder is given as an undercut portion. The forced ejection may, however, produce flash or burr on the mold product. The flash or burr may come into contact with the user's fingers when the tampon is used, and may undesirably raise uncomfortableness to the user.

### DISCLOSURE OF THE INVENTION

It is therefore an object of the present invention to provide a tampon applicator successfully solving the uncomfortableness when the tampon is used, by suppressing the flash or burr from being produced in the process of injection molding.

A first aspect of the present invention is summarized as a tampon applicator which includes: an outer cylinder housing therein an absorber, provided at one end thereof with an ejection opening through which the absorber is ejected, and provided at the other end thereof with a grip cylinder; and an inner cylinder inserted into the grip cylinder, and is capable of ejecting the absorber out through the ejection opening, when moved into the outer cylinder. The grip cylinder has a plurality of annular projections formed around the entire circumference and arranged in a row in the axial direction thereof, and the annular projections are formed so as to gradually reduce the height above the outer circumferential surface of the grip cylinder, towards the inner-cylinder-insertion side of the grip cylinder.

A second aspect of the present invention is summarized as the tampon applicator according to the first aspect. The plurality of annular projections arranged in a row are formed at different pitches between every adjacent annular projections.

A third aspect of the present invention is summarized as the tampon applicator according to the first aspect. Each annular projection has an apical surface laid in parallel with the outer circumferential surface of the grip cylinder, a vertical surface extending from the inner-cylinder-insertion side of the apical surface vertically down to the outer circumferential surface of the grip cylinder, and an inclined surface sloping from the absorber-housing side of the apical surface down to the outer circumferential surface of the grip cylinder.

A fourth aspect of the present invention is summarized as the tampon applicator according to the first aspect. Each annular projection is composed of a plurality of mountain-like projections each having an arciform apical surface.

According to the present invention, since the annular projections of the grip cylinder are formed so as to gradually reduce the height thereof towards the inner-cylinder-insertion side of the grip cylinder, so that the tampon applicator may smoothly be ejected from the die in the process of injection molding. Accordingly, the flash or burr may be suppressed from being produced, and uncomfortableness when the tampon is used may be relieved. In addition, since the projections are different in height, the tampon may be used under easier control of force.

There is another known problem of the conventional tampon applicator, such that the anti-slipping projections formed on the grip cylinder are not smooth in touch, and raise uncomfortableness to the fingers. There is still another problem in that the anti-slipping projections may even fail in making contact with the fingers, and consequently make the user uneasy in the process of handling.

It is, therefore, still another object to provide a tampon applicator ensuring a comfortable touch of the grip cylinder, and capable of exactly bringing the anti-slipping portions into contact with the fingers.

A fifth aspect of the present invention is summarized as a tampon applicator which includes: an outer cylinder housing therein an absorber, provided at one end thereof with an ejection opening through which the absorber is ejected, and provided at the other end thereof with a grip cylinder; and an inner cylinder inserted into the grip cylinder, and is capable of ejecting the absorber out through the ejection opening, when moved into the outer cylinder. The grip cylinder has a plurality of projection series formed thereon and arranged in a row in the axial direction thereof. Each projection series is composed of four projections which rise up from the outer circumferential surface of the grip cylinder at four positions in the circumferential direction thereof, each projection having a mountain-like sectional geometry with an arciform apex as viewed in the radial direction of the grip cylinder; and four base surfaces composed of the outer circumferential surface of the grip cylinder, and provided between every adjacent projections.

A sixth aspect of the present invention is summarized as the tampon applicator according to the fifth aspect. The plurality of projection series arranged in a row are formed so as to gradually reduce the height above the outer circumferential surface of the grip cylinder, towards the inner-cylinder-insertion side of the grip cylinder.

A seventh aspect of the present invention is summarized as the tampon applicator according to the fifth aspect. The plurality of projection series arranged in a row are formed at different pitches between every adjacent projection series.

An eighth aspect of the present invention is summarized as the tampon applicator according to the fifth aspect. Each projection has an apical surface laid in parallel with the outer circumferential surface of the grip cylinder, a vertical surface extending from the inner-cylinder-insertion side of the apical surface vertically down to the outer circumferential surface of the grip cylinder, and an inclined surface sloping from the absorber-housing side of the apical surface down to the outer circumferential surface of the grip cylinder.

According to the present invention, the grip cylinder, which is provided with the mountain-like projections in the projection series, is comfortable in touch. Accordingly, uncomfortableness in the process of handling may be avoidable. Since the projections are formed at four positions in the circumferential direction, the fingers may surely be brought into contact with the projections. As a consequence, uneasiness in the process of handling may be relieved.

### BRIEF DESCRIPTION OF DRAWINGS

FIG. 1 is a front elevation illustrating an entire view of a tampon applicator according to a first embodiment of the present invention;
FIG. 2 is an front elevation illustrating an enlarged view of portion "A" in FIG. 1;
FIG. 3 is a sectional view taken along line B-B in FIG. 2;
FIG. 4 is a sectional view illustrating a geometrical relation of the annular projections;
FIG. 5 is a sectional view illustrating a geometry of the one annular projection;
FIG. 6 is a graph illustrating a range of inclination of the inclined surfaces of the annular projections;
FIG. 7 is a front elevation illustrating an entire view of a tampon applicator according to a second embodiment of the present invention;
FIG. 8 is a front elevation illustrating an enlarged view of portion "A1" in FIG. 7;
FIG. 9 is a sectional view taken along line B1-B1 in FIG. 8;
FIG. 10 is a sectional view illustrating a geometrical relation of the projections and base surfaces in the projection series;
FIG. 11 is a sectional view illustrating a geometrical relation of the projection series;
FIG. 12 is a sectional view illustrating the geometry of the projection series;
FIG. 13 is a graph illustrating a range of inclination of the inclined surfaces of the projection series

### PREFERRED EMBODIMENTS FOR CARRYING OUT THE INVENTION

### (First Embodiment)

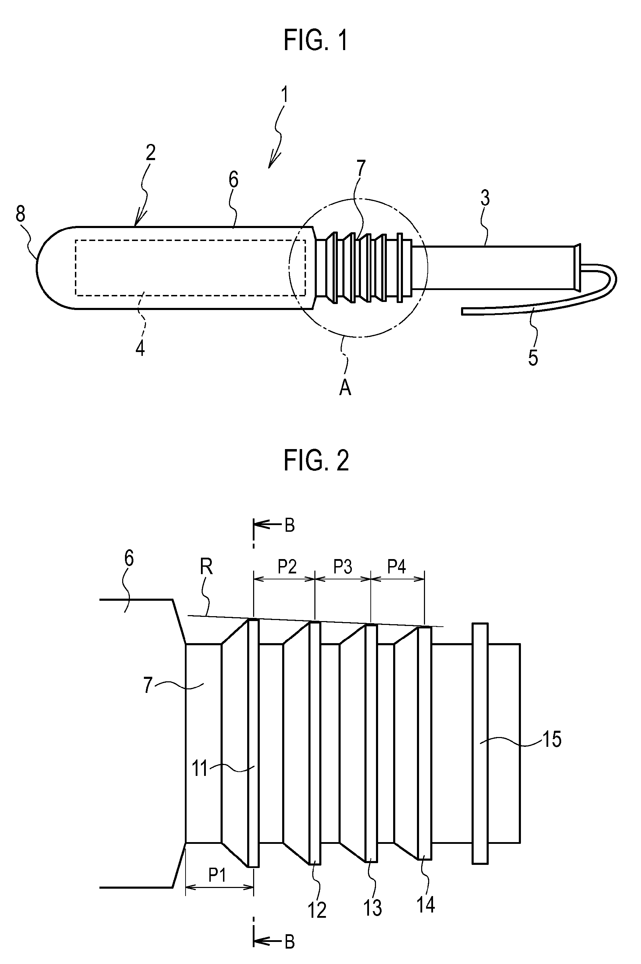

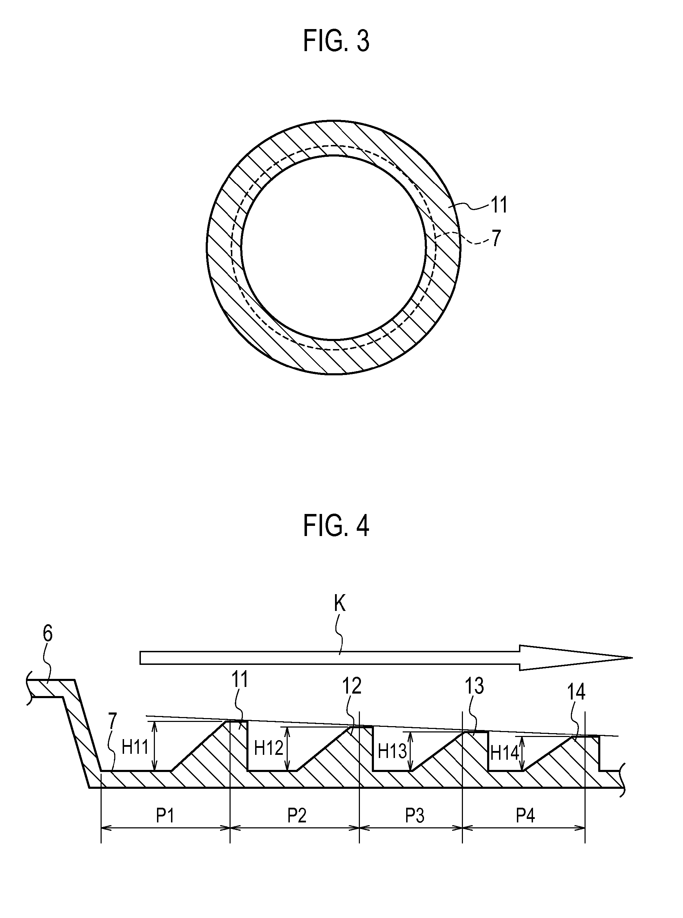

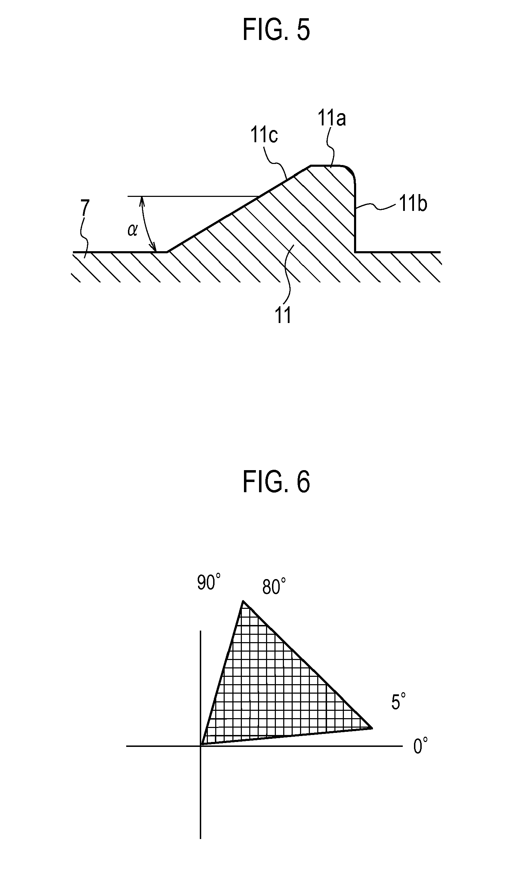

FIG. 1 is a front elevation illustrating an entire view of a tampon applicator according to a first embodiment of the present invention. FIG. 2 is an front elevation illustrating an enlarged view of portion "A" in FIG. 1. FIG. 3 is a sectional view taken along line B-B in FIG. 2. FIG. 4 is a sectional view illustrating a geometrical relation of the annular projections. FIG. 5 is a sectional view illustrating a geometry of one annular projection. FIG. 6 is a graph illustrating a range of degree of inclination a of the inclined surfaces of the annular projections.

A tampon applicator 1 of this embodiment has an outer cylinder 2 and an inner cylinder 3. Entire portions of these outer cylinder 2 and the inner cylinder 3 are respectively composed of a polyolefin such as polyethylene, polypropylene or the like.

In the outer cylinder 2, an absorber 4 is housed. At the base end of the absorber 4, a drawstring 5 is connected. The drawstring 5 extends from the base end of the absorber 4, and passes through the inner cylinder 3. The end portion of the inserted drawstring 5 is drawn out from the inner cylinder 3. The absorber 4 in use may be taken out from the body, by drawing the drawstring 5.

The end portion of the outer cylinder 2 is configured as a absorber-housing cylinder component 6 having a large diameter. The base end of the outer cylinder 2 is configured as a grip cylinder 7 having a small diameter. The end portion of the inner cylinder 3 is inserted into the grip cylinder 7. The end face of the inserted inner cylinder 3 is opposed to the absorber 4. The end portion of the absorber-housing cylinder component 6 is configured as an ejection opening. The ejection opening is connectively provided with petal-like components 8. The petal-like components 8 are normally closed at the edge of the ejection opening, and is opened while being stretched by the absorber 4, when the absorber 4 is ejected by the inner cylinder 3. In this way, the absorber 4 is ejected from the absorber-housing cylinder component 6 and may be inserted into the body. The grip cylinder 7 serves as a grip held by the fingers during handling for moving the inner cylinder 3.

As illustrated in FIG. 2, there are a plurality of annular projections 11, 12, 13 and 14 formed in a row on the outer circumference of the grip cylinder 7. Each of the annular projections 11, 12, 13 and 14 is formed over the entire circumference of the grip cylinder 7, as illustrated in FIG. 3. Each of the annular projections 11, 12, 13 and 14 forms a ring without being disconnected in the circumferential direction.

The annular projections 11, 12, 13 and 14 formed in a row function as a non-slip, when brought into contact with the user's fingers. Reference numeral 15 denotes a hitch formed on the inner-cylinder-insertion side of the grip cylinder 7. Also the hitch 15 is formed in a ring form similarly to the annular projections 11, 12, 13 and 14. The hitch 15 has a diameter larger than that of the adjacent annular projection 14, so as to catch the user's fingers.

The annular projections 11, 12, 13 and 14 in a row are formed so as to reduce the height above the outer circumferential surface of the grip cylinder 7, towards the insertion side where the inner cylinder 3 is inserted into the grip cylinder 7. More specifically, as illustrated in FIG. 2, the annular projection 11 on the side of the absorber-housing cylinder component 6 has a largest diameter, whereas the diameter gradually reduces as the position departs more largely from the absorber-housing cylinder component 6, in the decreasing order of the annular projections 12, 13 and 14. As a consequence, as illustrated in FIG. 2, apical line R connecting the apexes of the annular projections 11, 12, 13 and 14 shows an inclination which slopes down towards the side on which the inner cylinder 3 is inserted.

The annular projections 11, 12, 13 and 14 in a row are arranged so as to gradually reduce the height above the outer circumferential surface of the grip cylinder 7 towards the side on which the inner cylinder 3 is inserted into the grip cylinder 7, or in other words, so as to gradually increase the height above the outer circumferential surface of the grip cylinder 7 towards the ejection opening side described later. As a consequence, as illustrated in FIG. 2, the apical line R connecting the apexes of the annular projections 11, 12, 13 and 14 has an inclination which slopes up towards the ejection opening side.

FIG. 4 comparatively illustrates the height of the annular projections 11, 12, 13 and 14. Defining now the height of the annular projection 11 measured from the grip cylinder 7 as H11, and similarly the height of the annular projection 12 as H12, the height of the annular projection 13 as H13, and the height of the annular projection 14 as H14, they are adjusted so as to satisfy H11>H12>H13>H14. For example, the height of the annular projections may vary at 0.05-mm pitches between every adjacent annular projections, such as H11=0.4 mm, H12=0.35 mm, H13=0.3 mm and H14=0.25 mm. In this case, height H11 of the annular projection 11 may be varied within the range from 0.1 to 10 mm, and more preferably from 0.2 to 1 mm, and heights H12, H13 and H14 of the other annular projections are determined so as to sequentially decrease from this value in this order.

In FIG. 4, arrow K indicates the direction in which a die used for injection molding is drawn. The annular projections 11, 12, 13 and 14 are made lower in this order in the direction K of drawing. Therefore, forced ejection in the process of mold releasing is no more necessary, and the flash or burr ascribable to the forced ejection may be avoidable.

In this embodiment, the annular projections 11, 12, 13 and 14 are formed so as to make the adjacent inter-annular-projection pitches different from each other. In FIG. 2 and FIG. 4, P1 represents the pitch between the base end of the absorber-housing cylinder component 6 and the annular projection 11, P2 represents the pitch between the annular projection 11 and the annular projection 12, P3 represents the pitch between the annular projection 12 and the annular projection 13, and P4 represents the pitch between the annular projection 13 and the annular projection 14.

In this embodiment, inter-annular-projection pitches P2, P3, and inter-annular-projection pitches P3, P4 are set to different values. One typical setting is such as P2=2.5 mm, P3=2.0 mm and P4=2.5 mm. In this case, non-adjacent, inter-annular-projection pitches (P2 and P4) may have the same value, so far as the adjacent inter-annular-projection pitches differ from each other.

Difference in the adjacent inter-annular-projection pitches may fall in the range preferably from 0.2 to 5 mm, and more preferably from 1.5 to 3 mm. The difference in the adjacent pitches smaller than 0.2 mm is not desirable, because the user may be hard to feel difference in the pitch when her fingers come into contact therewith. Also the range of difference exceeding 5 mm is not desirable, because the grip cylinder 7 will be unnecessarily long. Pitch P1 between the absorber-housing cylinder component 6 and the annular projection 11 may be set depending on the length of the grip cylinder 7, typically as P1 =3.0 mm.

By varying the adjacent inter-annular-projection pitches as described in the above, the user may feel desirable grip when she holds the grip cylinder 7.

The annular projections 11, 12, 13 and 14 in this embodiment are formed to have a trapezoidal section as viewed in the axial direction. More specifically, as illustrated in FIG. 5, the annular projection 11 has an apical surface 11a laid in parallel with the outer circumferential surface of the grip cylinder 7 (the same will apply also to the other annular projections 12, 13 and 14). From the apical surface 11a, a vertical surface 11b and an inclined surface 11c connectively extend to form the trapezoidal section.

The vertical surface 11b is formed so as to extend from the end of the apical surface 11a, on the side thereof where the inner cylinder 3 is inserted (on the right side in FIG. 5), vertically down to the outer circumferential surface of the grip cylinder 7. The inclined surface 11c is formed so as to extend from the end of the apical surface 11a, on the side thereof where the absorber 4 is housed (absorber-housing cylinder component 6, on the left side in FIG. 5), while sloping down to the outer circumferential surface of the grip cylinder 7.

The width of the apical surface 11a of the annular projection 11 is set preferably to 0.1 to 5 mm, and more preferably to 0.2 to 2 mm (the same will apply also to the apical surfaces of the other annular projections 12, 13 and 14). If the width of the apical surface 11a exceeds these ranges, the flash or burr may undesirably be produced in the process of mold releasing in injection molding. The angle of downward inclination of the inclined surface 11c is preferably set in relation with the height of the absorber-housing cylinder component 6.

More specifically, as illustrated in FIG. 6, when the height of the annular projection 11 is set to a half-height of the absorber-housing cylinder component 6, angle α of the inclined surface 11c away from the outer circumferential surface of the grip cylinder 7 is adjusted preferably to the range from 5 to 80°, and preferably from 20 to 60°. When the angle of the inclined surface 11c is 90°, the flash or burr may be more likely to generate in the process of mold releasing in injection molding. The angle of the inclined surface 11c is set similarly also to the other annular projections 12, 13 and 14.

In the embodiment described in the above, since the height of the plurality of annular projections 11, 12, 13 and 14 are set so as to gradually reduce towards the side on which the inner cylinder 3 is inserted into the grip cylinder 7, so that the mold releasing may exactly be carried out. As a consequence, the flash and burr are suppressed from being produced. In addition, since the adjacent inter-annular-projection pitches are made different from each other, so that the sense of hitch felt by the user's fingers may be enhanced. Accordingly the feeling of grip may be improved.

### (Second Embodiment)

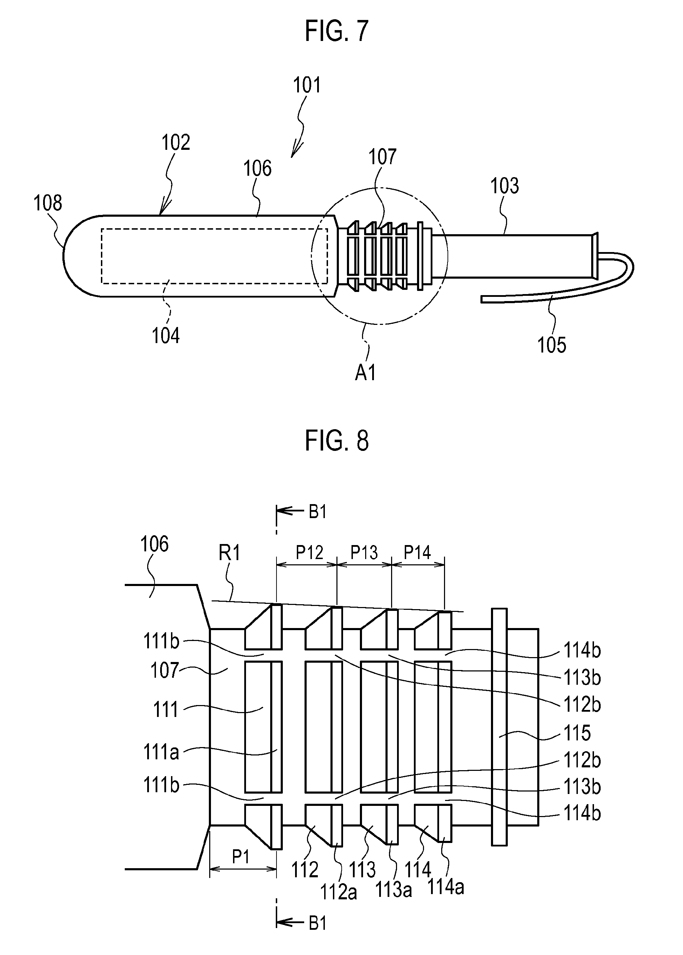

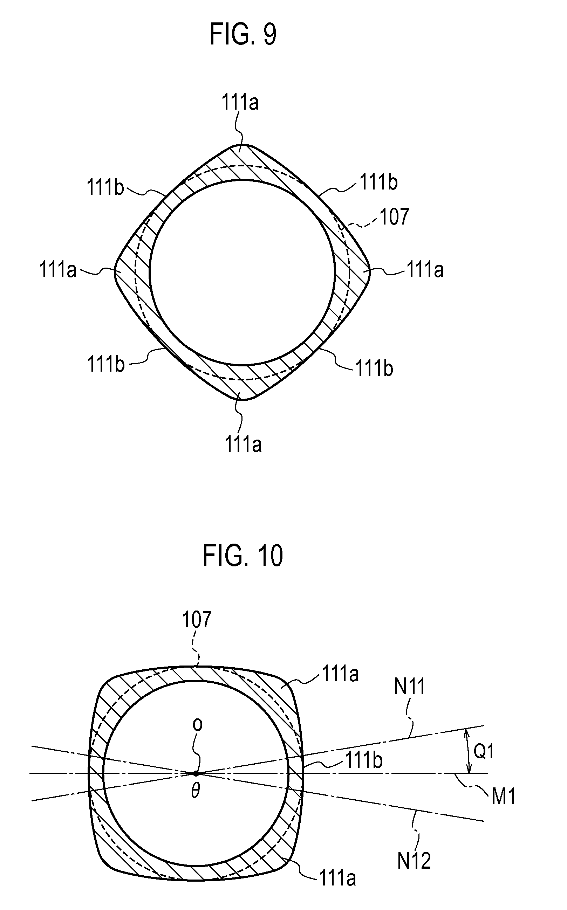

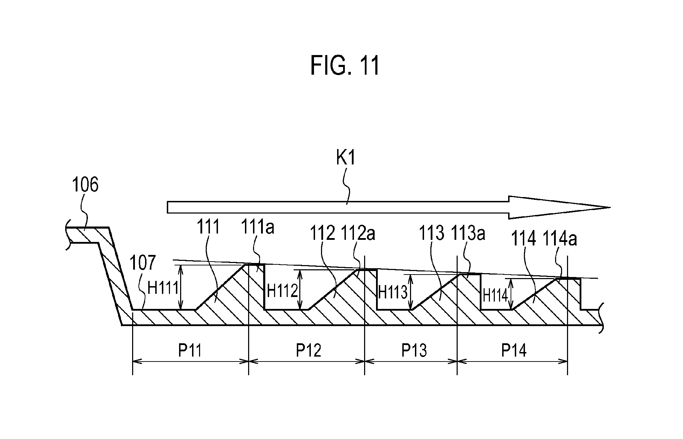

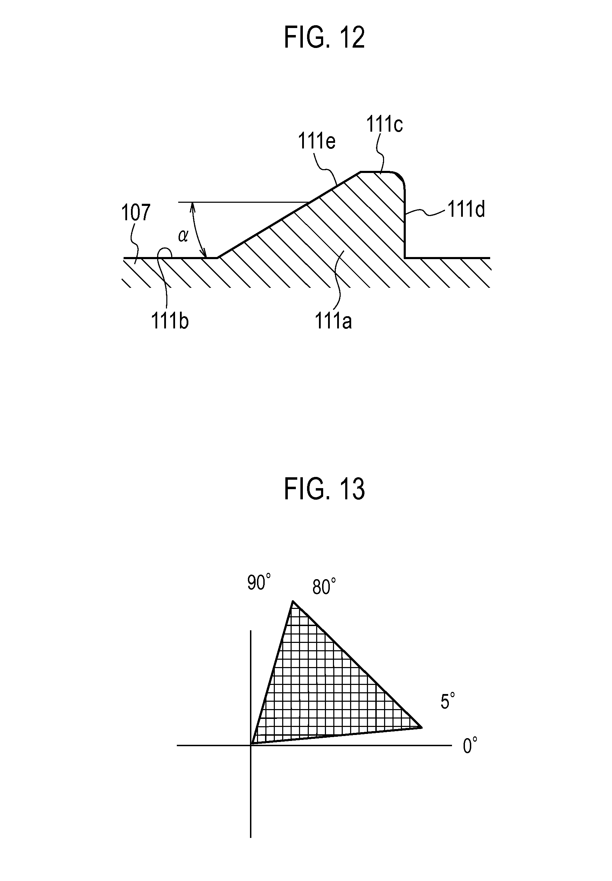

FIG. 7 is a front elevation illustrating an entire view of a tampon applicator according to a second embodiment of the present invention. FIG. 8 is a front elevation illustrating an enlarged view of portion "A1" in FIG. 7. FIG. 9 is a sectional view taken along line B1-B1 in FIG. 8. FIG. 10 is a sectional view illustrating the projection series taken at the same position with FIG. 9. FIG. 11 is a sectional view illustrating a geometrical relation of the projection series. FIG. 12 is a sectional view illustrating the geometry of one projection in the projection series. FIG. 13 is a graph illustrating a range of degree of inclination α1 of the inclined surface of the projection;

As illustrated in FIG. 8, there are a plurality of annular projection series 111, 112, 113 and 114 formed in a row on the outer circumference of the grip cylinder 107. The plurality of projections 111, 112, 113 and 114 in a row function as a non-slip, when brought into contact with the user's fingers. Reference numeral 115 denotes a hitch formed to the grip cylinder 107 on the side thereof where the inner cylinder 103 is inserted. The hitch 115 has a diameter larger than that of the adjacent annular projection 114, so as to catch the user's fingers.

The individual projection series 111, 112, 113 and 114 are composed of four projections 111a, 112a, 113a and 114a and four base surfaces 111b, 112b, 113b and 114b, respectively.

FIG. 9 illustrates a section of the projection series 111 as viewed in the radial direction. The same will apply also to the other projection series 112,113 and 114. Four projections 111a in the projection series 111 rise up at four positions in the circumferential direction on the outer circumferential surface of the grip cylinder 107. In this embodiment, the projections 111a rise up at four positions defined by quadrisection of the circumference. Each projection 111a has a mountain-like geometry with an arciform apex. The arciform profile of the apex ensures gentle touch to the fingers. The base surfaces 111b are located between the projections 111a. The base surfaces 111b are configured by the same surface with the outer circumferential surface of the grip cylinder 107. Since the grip cylinder 107 is given as a round cylinder, so that also the base surfaces 111b, which are identical to the outer circumferential surface of the grip cylinder 107, are given with an arciform profile. Accordingly, the entire portion of the projection series 111 may ensure a gentle touch to the fingers.

FIG. 10 illustrates a geometrical relation of arrangement of the projections 111a and the base surfaces 111b in the projection series 111. The same will apply also to the other projection series 112,113 and 114. The apex of each projection 111a is formed to as high as 0.1 to 10 mm, and more preferably 0.2 to 1 mm, above the outer circumferential surface of the grip cylinder 107. In this case, the diameter of the grip cylinder 107 is preferably 4 to 25 mm, and more preferably 13 to 16 mm.

In FIG. 10, line M1 represents a center line which passes through the center point O of the grip cylinder 107, and lines N11 and N12 determine the width of the base surfaces 111b, or specify the regions where the projections 111a are discontinued. Lines N11 and N12 are located on both sides while placing center line M1 in between. The angle formed between one line N11 and center line M1, or a region Q1 which corresponds to a half of one base surface 111b, preferably ranges from 1 to 40°, and more preferably from 5 to 15°.

By composing the projection series 111 with these sorts of projections 111a and base surfaces 111b, not only a smooth touch, but also an anti-slipping performance may be obtained. In addition, a part of resin material composing the projections 111a may move to the base surfaces 111b in the process of mold releasing in injection molding. As a consequence, the flash may be suppressed from occurring at the projections 111a. Note that the moved resin recover its original position (projections 111a) after the mold releasing.

The projections 111a, 112a, 113a and 114a and the base surfaces 111b, 112b, 113b and 114b illustrated in FIG. 8 are arranged at the same positions in the radial direction of the grip cylinder 107 in the plurality of projection series 111, 112, 113 and 114. The arrangement is, however, not limited thereto, instead allowing arrangement of those at different positions in the radial direction.

The projections 111a, 112a, 113a and 114a of the plurality of projection series 111, 112, 113 and 114 are formed so as to gradually reduce the height above the outer circumferential surface of the grip cylinder 107, towards the side on which the inner cylinder 103 is inserted into the grip cylinder 107. More specifically, as illustrated in FIG. 8, the projections 111a of the projection series 111 located on the absorber-housing cylinder component 106 side has a largest height, whereas the height gradually reduces as the position departs more largely from the absorber-housing cylinder component 106. More specifically, as viewed in the order of the projection series 111, 112, 113 and 114, the height of the projections 111a, 112a, 113a and 114a decreases. As a consequence, as illustrated in FIG. 8, apical line R1 connecting the apexes of the projections 111a, 112a, 113a and 114a shows an inclination which slopes down towards the side on which the inner cylinder 103 is inserted.

The projections 111a, 112a, 113a and 114a of the plurality of projection series 111, 112, 113 and 114 are arranged so as to gradually reduce the height above the outer circumferential surface of the grip cylinder 107 towards the side on which the inner cylinder 103 is inserted into the grip cylinder 107, or in other words, so as to gradually increase the height above the outer circumferential surface of the grip cylinder 107 towards the ejection opening side described later. As a consequence, as illustrated in FIG. 8, the apical line R1 connecting the apexes of the projections 111a, 112a, 113a and 114a has an inclination which slopes up towards the ejection opening side.

FIG. 11 comparatively illustrates the height of the projections 111a, 112a, 113a and 114a of the projection series 111, 112, 113 and 114. Defining now the height of the projections 111a of the projection series 111 as H111, the height of the projections 112a of the projection series 112 as H112, the height of the projections 113a of the projection series 113 as H113, and the height of the projections 114a of the projection series 114 as H114, they are adjusted so as to satisfy H111>H112>H113>H114. For example, the height of the projections may vary at 0.05-mm pitches between every adjacent annular projections, such as H111 =0.4 mm, H112=0.35 mm, H113=0.3 mm and H114=0.25 mm. In this case, height H111 of the projections 111a may be varied within the range from 0.1 to 10 mm, and more preferably from 0.2 to 1 mm, and heights H112, H113 and H114 of the other projections 112a, 113a and 114a are determined so as to sequentially decrease from this value in this order.

In FIG. 11, arrow K1 indicates the direction in which a die used for injection molding is drawn. The projection series 111, 112, 113 and 114 are made lower in this order in the direction K1 of drawing. Therefore, forced ejection in the process of mold releasing is no more necessary, and the flash or burr ascribable to the forced ejection may be avoidable.

In this embodiment, the projections 111a, 112a, 113a and 114a of the projection series 111, 112, 113 and 114 are formed so as to make the adjacent inter-projection-series pitches different from each other. In FIG. 8 and FIG. 11, P11 represents the pitch between the base end of the absorber-housing cylinder component 106 and the projections 111a of the projection series 111, P12 represents the pitch between the projections 111a of the projection series 111 and the projections 112a of the projection series 112, P13 represents the pitch between the projections 112a of the projection series 112 and the projections 113a of the projection series 113, and P14 represents the pitch between the projections 113a of the projection series 113 and the projections 114a of the projection series 114.

In this embodiment, the inter-projection-series pitches P12, P13, and the inter-projection-series pitches P13, P14 are set to different values. One typical setting is such as P12=2.5 mm, P13=2.0 mm and P14=2.5 mm. In this case, non-adjacent, inter-projection-series pitches (P12 and P14) may have the same value, so far as the adjacent inter-projection-series pitches differ from each other.

Difference in the adjacent inter-projection-series pitches may fall in the range preferably from 0.2 to 5 mm, and more preferably from 1.5 to 3 mm. The difference in the adjacent pitches smaller than 0.2 mm is not desirable, because the user may be hard to feel difference in the pitch when her fingers come into contact therewith. Also the range of difference exceeding 5 mm is not desirable, because the grip cylinder 107 will be unnecessarily long. The pitch P11between the absorber-housing cylinder component 106 and the projections 111a of the projection series 111 may be set depending on the length of the grip cylinder 107, typically as P11=3.0 mm.

By varying the adjacent inter-projection-series pitches as described in the above, the user may feel desirable grip when she holds the grip cylinder 107.

The projections 111a, 112a, 113a and 114a of the projection series 111, 112, 113 and 114 in this embodiment are formed to have a trapezoidal section as viewed in the axial direction. More specifically, as illustrated in FIG. 12, the projection 111a of the projection series 111 has an apical surface 111c laid in parallel with the outer circumferential surface of the grip cylinder 107, and a vertical surface 111d and an inclined surface 111e connectively extend from the apical surface 111c, so as to form the trapezoidal section as viewed in the axial direction (the same will apply also to the other projection series 112, 113 and 114). The vertical surface 111d is formed so as to extend from the end of the apical surface 111c, on the side thereof where the inner cylinder 103 is inserted (on the right side in FIG. 12), vertically down to the outer circumferential surface of the grip cylinder 107. The inclined surface 111e is formed so as to extend from the end of the apical surface 111c, on the side thereof where the absorber 104 is housed (absorber-housing cylinder component 106, on the left side in FIG. 12), while sloping down to the outer circumferential surface of the grip cylinder 107.

The width of the apical surface 111c of the projections 111a of the projection series 111 is set preferably to 0.1 to 5 mm, and more preferably to 0.2 to 2 mm (the same will apply also to the apical surfaces of the projections of the other projection series 112, 113 and 114). If the width of the apical surface 111c exceeds these ranges, the flash or burr may undesirably be produced in the process of mold releasing in injection molding. The angle of downward inclination of the inclined surface 111e is preferably set in relation with the height of the absorber-housing cylinder component 106. More specifically, as illustrated in FIG. 13, when the height of the projections 111a of the projection series 111 is set to a half-height of the absorber-housing cylinder component 106, angle α1 of the inclined surface 111e away from the outer circumferential surface of the grip cylinder 107 is adjusted preferably to the range from 5 to 80°, and preferably from 20 to 60°. When the angle of the inclined surface 111e is 90°, the flash or burr may be more likely to generate in the process of mold releasing in injection molding. The angle of the inclined surface 111e is set similarly also to the projections 112a, 113a and 114a of the other projection series 112, 113 and 114.

Since the mountain-like projections 111a, 112a, 113a and 114a are provided to the projection series 111, 112, 113 and 114 formed to the grip cylinder 107 in the above-described embodiment, so that the grip cylinder 107 ensures a desirable touch to the fingers. As a consequence, the tampon applicator does not make the user feel uncomfortable in handling. In addition, since the projections 111a, 112a, 113a and 114a are formed at four positions in the circumferential direction, so that the fingers may exactly be brought into contact therewith. As a consequence, uneasiness of the user in the handling may be relieved.

This application is based upon and claims the benefit of priority from the prior Japanese Patent Application No. JP 2008-081950, filed on March 26, 2008;and Japanese Patent Application No. JP 2008-081964, filed on March 26, 2008; the entire contents of which are incorporated herein by reference.

### INDUSTRIAL APPLICABILITY

As has been described in the above, the tampon applicator of the present invention is useful, because uncomfortableness of the user possibly felt when she uses it may be relieved, by suppressing the flash or burr from generating on the grip cylinder in the process of injection molding. The tampon applicator of the present invention is therefore useful.

The tampon applicator of the present invention has mountain-like projections provided to the projection series formed to the grip cylinder, so that the tampon applicator is desirable in touch, without raising uncomfortableness to the user. Since the projections are formed at four positions in the circumferential direction, the fingers may exactly be brought into contact with the projections, and thereby uneasiness in the process of handling may be relieved. The tampon applicator of the present invention is therefore useful.

## Claims

1. A tampon applicator comprising:
an outer cylinder housing therein an absorber, provided at one end thereof with an ejection opening through which the absorber is ejected, and provided at the other end thereof with a grip cylinder; and
an inner cylinder inserted into the grip cylinder, and is capable of ejecting the absorber out through the ejection opening, when moved into the outer cylinder, wherein
the grip cylinder has a plurality of annular projections formed around the circumference and arranged in a row in the axial direction thereof, the annular projections being formed so as to gradually reduce the height above the outer circumferential surface of the grip cylinder, towards the inner-cylinder-insertion side on which the inner cylinder is inserted into the grip cylinder.

2. The tampon applicator according to claim 1, wherein
the plurality of annular projections arranged in a row are formed at different pitches between every adjacent annular projections.

3. The tampon applicator according to claim 1, wherein
each annular projection has an apical surface laid in parallel with the outer circumferential surface of the grip cylinder, a vertical surface extending from the inner-cylinder-insertion side of the apical surface vertically down to the outer circumferential surface of the grip cylinder, and an inclined surface sloping from the absorber-housing side of the apical surface down to the outer circumferential surface of the grip cylinder.

4. The tampon applicator according to claim 1, wherein
each annular projection is composed of a plurality of mountain-like projections each having an arciform apical surface.

5. A tampon applicator comprising:
an outer cylinder housing therein an absorber, provided at one end thereof with an ejection opening through which the absorber is ejected, and provided at the other end thereof with a grip cylinder; and
an inner cylinder inserted into the grip cylinder, and is capable of ejecting the absorber out through the ejection opening, when moved into the outer cylinder, wherein
the grip cylinder has a plurality of projection series formed thereon and arranged in a row in the axial direction thereof, each projection series being composed of four projections which rise up from the outer circumferential surface of the grip cylinder at four positions in the circumferential direction thereof, each projection having a mountain-like sectional geometry with an arciform apex as viewed in the radial direction of the grip cylinder; and four base surfaces composed of the outer circumferential surface of the grip cylinder, and provided between every adjacent projections.

6. The tampon applicator according to claim 5, wherein
the plurality of projection series arranged in a row are formed so as to gradually reduce the height above the outer circumferential surface of the grip cylinder, towards the inner-cylinder-insertion side of the grip cylinder.

7. The tampon applicator according to claim 5, wherein
the plurality of projection series arranged in a row are formed at different pitches between every adjacent projection series.

8. The tampon applicator according to claim 5, wherein
each projection has an apical surface laid in parallel with the outer circumferential surface of the grip cylinder, a vertical surface extending from the inner-cylinder-insertion side of the apical surface vertically down to the outer circumferential surface of the grip cylinder, and an inclined surface sloping from the absorber-housing side of the apical surface down to the outer circumferential surface of the grip cylinder.
